Europäisches Patentamt

European Patent Office

Office européen des brevets

(19)

(11) Publication number: **0 369 728**
**A1**

## (12) EUROPEAN PATENT APPLICATION

(21) Application number: **89311754.9**

(22) Date of filing: **14.11.89**

(51) Int. Cl.⁵: **C07C 17/32, C07C 22/04**

(30) Priority: **14.11.88 US 270621**

(43) Date of publication of application:
**23.05.90 Bulletin 90/21**

(84) Designated Contracting States:
**CH DE FR GB IT LI**

(71) Applicant: **ETHYL CORPORATION**
**451 Florida Boulevard**
**Baton Rouge, LA 70801(US)**

(72) Inventor: **Stahly, Barbara Clack**
**517 Corsair Drive**
**Baton Rouge Louisiana 70810(US)**
Inventor: **Benage, Brigitte**
**11580 Perkins Road No. 28**
**Baton Rouge Louisiana 70810(US)**

(74) Representative: **Collier, Jeremy Austin Grey et al**
**J.A.Kemp & Co. 14, South Square Gray's Inn**
**London WC1R 5EU(GB)**

(54) **Haloalkylation of aromatic compounds.**

(57) The co-formation of diarylalkane by-product in the haloalkylation of an aromatic compound with an ether corresponding to the formula R-O-R′, wherein R is an α-haloalkyl group containing at least two carbons and R′ is R or alkyl, is minimized by conducting the reaction with agitation in the presence of both hydrogen sulfate and a hydrogen halide.

EP 0 369 728 A1

## HALOALKYLATION OF AROMATIC COMPOUNDS

This invention relates to a process for haloalkylating aromatic compounds to form 1-halo-1-arylalkanes.

As disclosed in March, Advanced Organic Chemistry, Second Edition, McGraw-Hill, New York, 1977, pp. 501-502; Olah, Friedel-Crafts and Related Reactions, Volume 2, Interscience Publishers, New York, 1963-1964, pp. 659-784; U. S. Patent 2,516,971 (Galitzenstein et al.); and the references cited therein, it is known that aromatic compounds can be haloalkylated by reacting them with a hydrogen halide and an appropriate aldehyde, or with an α-haloalkyl ether or an α-haloalkyl alkyl ether, in the presence of a Lewis acid or a proton acid as a catalyst, most commonly in the presence of zinc chloride.

The haloalkylations utilizing formaldehyde or a formaldehyde-derived ether have been successfully employed in providing fairly high yields of 1-halo-1-arylalkanes, and reasonably high yields of 1-halo-1-arylalkanes have sometimes also been obtained from haloalkylations utilizing higher aldehydes or ethers derived from them. However, it has frequently not been found possible to provide commercially acceptable yields of 1-halo-1-arylalkane from the higher aldehydes and ethers. There has been too much co-formation of diarylalkane by-product. It would be desirable to find a way of increasing the 1-halo-1-arylalkane yields obtainable from such processes to provide a more economical method of preparing, e.g., the 1-halo-1-(4-alkylphenyl)alkanes used in known processes, such as those of U.S. Patent 4,536,595 (Gardano et al.), Canadian Patent 1,197,254 (Francalanci et al.), British Patent 1,560,082 (Dynamit Nobel), Czechoslovakian Certificate of Authorship 219,752 (Palecek et al.), and Japanese Kokai 47-39050 (Miyatake et al.) and 52-111536 (Tokutake).

An object of this invention is to provide a novel process for haloalkylating an aromatic compound, more particularly a monoalkylaromatic hydrocarbon, to form a 1-halo-1-arylalkane. The new process minimizes co-formation of a diarylalkane by-product.

1-Halo-1-arylalkanes are useful as chemical intermediates.

The process of the invention comprises reacting one molar proportion of a monoalkylaromatic hydrocarbon with at least one molar proportion of an ether corresponding to the formula R-O-R$'$, wherein R is an alpha-haloalkyl group containing at least two carbons and R$'$ is R or alkyl, with agitation at a temperature in the range of -35°C to 0°C in the presence of at least one molar proportion of a hydrogen halide and 2-15 molar proportions of hydrogen sulfate to haloalkylate the monoalkylaromatic hydrocarbon while minimizing the co-formation of diarylalkane by-product.

In the 1-halo-1-arylalkane product, the aromatic nucleus carries the introduced haloalkyl radical usually in the para position with respect to the alkyl substituent in the starting monoalkylaromatic hydrocarbon, but in a minor proportion of the product, the said radicals may be in ortho relationship. The monoalkylaromatic hydrocarbon may be, for example, 1-methylnaphthalene, 2-methylnaphthalene, 9-methylanthracene, 9-butylanthracene, 9-dodecylanthracene, or a monoalkylbenzene, e.g., the methyl-, ethyl-, propyl-, isobutyl-, sec-butyl-, t-butyl-, isopentyl-, t-pentyl-, and hexylbenzenes. The most preferred aromatic compounds are the monoalkylbenzenes wherein the alkyl group contains 1-5 carbons.

The ether which is reacted with the aromatic hydrocarbon is an ether corresponding to the formula R-O-R$'$, wherein R is an alpha-haloalkyl group containing at least two carbons, preferably 2-20 carbons, and most preferably 2-6 carbons, and R$'$ and R or an alkyl group which preferably contains 1-20 carbons, most preferably 1-6 carbons. The halo substituent is preferably chloro or bromo.

Exemplary of the ethers which may be employed are α-chloroethyl ether (also known as chloroethyl ether, 1-chloroethyl ethers, bis(1-chloroethyl) ether, or di(1-chloroethyl) ether), α-chloropropyl ether, α-chlorobutyl ether, α-chloropentyl ether, α-chlorohexyl ether, α-chlorodecyl ether, α-chlorododecyl ether, α-chloropentadecyl ether, α-chlorooctadecyl ether, α-chloroeicosyl ether, α-chloroethyl methyl ether, α-chloroethyl ethyl ether, α-chloroethyl propyl ether, α-chlorobutyl butyl ether, α-chloropentyl methyl ether, α-chlorohexyl hexyl ether, and the corresponding bromo and iodo compounds. The preferred ethers are the α-chloroalkyl ethers, such as α-chloroethyl ether, and the α-chloroalkyl alkyl ethers, such as α-chloroethyl methyl ether and α-chloroethyl ethyl ether.

When not already available, the ethers may be formed by conventional techniques of reacting the appropriate hydrogen halide with the appropriate aldehyde and, when a haloalkyl alkyl ether is desired, also with the appropriate alcohol to form the desired ether and water. The water may or may not be removed from the reaction product before the ether is used in the haloalkylation process, but it is generally preferred to remove any water that would cause the water content of the haloalkylation reaction mixture to exceed 15% by weight of the catalyst used.

The amount of ether employed in the haloalkylation reaction may be as small as the stoichiometric amount, i.e., the amount which provides one R group per molecule of aromatic hydrocarbon. However, it is generally preferred to employ an amount that provides at least two R groups per molecule of aromatic compound. There does not appear to be any maximum to the amount of ether that may be used other than the maximum that economics permit.

As in known processes, the haloalkylation is conducted in the presence of an acid catalyst, preferably hydrogen sulfate. In order to avoid the presence of too much water in the reaction mixture, as well as to take advantage of commercially-available materials, the hydrogen sulfate is generally introduced in the form of 88-98% sulfuric acid. The amount employed is generally such as to provide at least one mol, preferably at least 2-6 mols, per mol of aromatic compound; and it ordinarily should not exceed 15 mols per mol of aromatic compound.

The hydrogen halide which is used in the reaction may be any hydrogen halide but is preferably hydrogen chloride or hydrogen bromide, most preferably hydrogen chloride. The amount of hydrogen halide is usually at least one equivalent, based on the amount of aromatic compound; and it is generally introduced by bubbling it through the reaction mixture or by pressurizing the reaction vessel with it.

Since improved yields of 1-halo-1-arylalkane are not obtained without it, the use of the hydrogen halide is critical -- a surprising factor, since the ether already contains the haloalkyl group which is to be attached to the aromatic hydrocarbon, and the reaction mixture already contains an acid catalyst. It would therefore have been thought that neither an additional halogen source nor additional acidity would be necessary.

The reaction is usually conducted at a bath temperature in the range of -35°C to +25°C, preferably -35°C to 0°C, in order to achieve the maximum advantages of the invention. The higher temperatures generally favor higher conversions, while the lower temperatures are apt to favor higher haloalkylation product/diarylalkane ratios.

The manner of combining the ingredients does not appear to be critical. For example, (1) the ether, which may be a pure ether or a crude ether contaminated with water and/or hydrogen halide, may be dissolved in the aromatic compound and added to the catalyst while bubbling hydrogen halide through the reaction mixture, (2) the catalyst may be added to such a pure or crude ether prior to the addition of the aromatic compound, (3) the pure or crude ether, the aromatic compound, and the catalyst may be combined in either fashion in a reaction vessel which is pressurized with the hydrogen halide.

The invention is useful as an alternative method of preparing 1-halo-1-arylalkanes from aromatic compounds that are known to be capable of providing high yields of such products by known haloalkylation techniques. However, it is particularly advantageous as a method of preparing 1-halo-1-arylalkanes from the less reactive aromatic hydrocarbons, such as monoalkylbenzenes, that have not previously been found to be capable of providing high yields of such products by haloalkylation processes other than halomethylations.

As is known, the products obtained by the process are useful as internal standards, intermediates for the preparation of monomers, detergents, or pharmaceuticals. When they are used as chemical intermediates, they may be subjected to the same reactions as have previously been used to convert them to desired products. For example, the 1-halo-1-phenylethanes can be dehydrohalogenated in any known manner to provide styrenes which can then be polymerized by known techniques.

A particularly interesting application of the 1-halo-1-(4-alkylphenyl)ethanes which are prepared in a preferred embodiment of the invention is as intermediates for the preparation of ibuprofen and related pharmaceuticals. When they are used in such applications, they may be converted to the desired products in any suitable manner. For example, they may be reacted with carbon monoxide in the presence of a carbonylation catalyst and then acidified to the corresponding propionic acids as in Gardano et al., Francalanci et al., or Dynamit Nobel; or they may be cyanated and then acidified to the corresponding propionic acids as in Palecek et al. or Tokutake. Another useful synthesis involves reacting the compounds with magnesium, carbonating the resultant Grignard reagent with carbon dioxide, and acidifying the carbonated product to the propionic acid as in Miyatake et al.

The following example is given to illustrate the invention and is not intended as a limitation thereof.

## EXAMPLE

A solution of 5 mL of isobutylbenzene and 10 mL of α-chloroethyl ether was added dropwise over a period of ten minutes to 10 mL of 93% sulfuric acid which had been cooled to a bath temperature of -17°C and through which anhydrous hydrogen chloride gas was bubbled. The bath temperature of -17°C and the bubbling of anhydrous hydrogen chloride through the vessel were continued while the reaction mixture was stirred for 90 minutes.

The reaction mixture was then added to 50 mL of ice water and stirred vigorously for 15 minutes, after which 25 mL of diethyl ether was added and the layers were separated. The organic layer was dried over sodium sulfate, filtered, and concentrated under reduced pressure. NMR spectroscopy using 1,1,2,2-tetrachloroethane as an internal standard showed a 49% recovery of isobutylbenzene, a 33% yield of 1-chloro-1-(4-isobutylphenyl)ethane, and a 4% yield of 1,1-bis(4-isobutylphenyl)ethane.

range of -35°C to 0°C in the presence of 2-6 molecular proportions of hydrogen sulfate and in the absence of more than 15% by weight of water, based on the weight of the hydrogen sulfate, while bubbling hydrogen chloride through the reaction mixture or pressurizing the reaction vessel with hydrogen chloride; the hydrogen sulfate being introduced in the form of 88-98% sulfuric acid.

## Claims

1. A process which comprises reacting one molar proportion of a monoalkylaromatic hydrocarbon with at least one molar proportion of an ether corresponding to the formula R-O-R', wherein R is an α-haloalkyl group containing at least two carbons and R' is R or alkyl, with agitation at a temperature in the range of -35°C to 0°C in the presence of at least one molar proportion of a hydrogen halide and 2-15 molar proportions of hydrogen sulfate to haloalkylate the monoalkylaromatic hydrocarbon while minimizing the co-formation of diarylalkane by-product.

2. The process of claim 1 wherein the monoalkylaromatic hydrocarbon is a monoalkylbenzene.

3. The process of claim 2 wherein the monoalkylbenzene is one in which the alkyl substituent contains 1-5 carbons.

4. The process of claim 1, 2 or 3 wherein the ether is one in which R is an α-haloalkyl group containing 2-20 carbons and R' is R or an alkyl group containing 1-20 carbons.

5. The process of claim 4 wherein the ether is one in which R is an α-chloroalkyl group containing 2-6 carbons and R' is R or an alkyl group containing 1-6 carbons.

6. The process of claim 4 wherein the ether is one in which R is an α-bromoalkyl group containing 2-6 carbons and R' is R or an alkyl group containing 1-6 carbons.

7. The process of any one of claims 1 to 6 wherein the amount of ether employed is such as to provide at least two R groups per molecule of aromatic compound.

8. The process of any one of claims 1 to 7 wherein the amount of hydrogen sulfate is 2-6 mols per mol of aromatic compound.

9. The process of any one of claims 1 to 8 wherein the reaction is conducted in the absence of more than 15% by weight of water, based on the weight of the hydrogen sulfate.

10. The process of claim 1 wherein one molecular proportion of isobutylbenzene is chloroethylated by reacting it with at least two molecular proportions of α-chloroethyl ether at a temperature in the

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A- 689 430 (A. BOEHRINGER et al.) * Claims * | 1 | C 07 C 17/32 C 07 C 22/04 |
| A | US-A-3 539 612 (R.C. TWEIT) * Example 8 * | | |

TECHNICAL FIELDS SEARCHED (Int. Cl.5)

C 07 C 17/00

The present search report has been drawn up for all claims

| Place of search | Date of completion of the search | Examiner |
|---|---|---|
| THE HAGUE | 05-02-1990 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)